(19) **Europäisches Patentamt**
**European Patent Office**
**Office européen des brevets**

(11) Publication number: **0 436 086 A1**

(12) # EUROPEAN PATENT APPLICATION

(21) Application number: **90121038.5**

(22) Date of filing: **02.11.90**

(51) Int. Cl.⁵: **C07K 13/00, C07K 15/00, G01N 33/574**

(30) Priority: **21.12.89 KR 8919150**

(43) Date of publication of application:
**10.07.91 Bulletin 91/28**

(84) Designated Contracting States:
**CH DE FR GB LI**

(71) Applicant: **KUKJE PHARMA IND. CO., LTD**
**648, Choji-dong, Ansan**
**Kyunggi-do(KR)**

(72) Inventor: **Nam,Young Woo**
**151 Yumgok-dong**
**Seocho-ku, Seoul(KR)**

(74) Representative: **Kraus, Walter, Dr. et al**
**Patentanwälte Kraus, Weisert & Partner**
**Thomas-Wimmer-Ring 15**
**W-8000 München 22(DE)**

(54) **Isolation of alpha-1-antitrypsin from human plasma and construction of a kit useful for detecting alpha 1-antitrypsin levels in blood.**

(57) There is described a method for purifying homogeneous form of $\alpha_1$-antitrypsin (AAT) using the three connected columns (Affi-Gel Blue I, Affi-Gel II, and DEAE-cellulose) after purifying it partially through ammonium sulfate fractionation (between 50 and 75%) and agarose gel electrophoresis as well as a corresponding kit for measuring levels of serum $\alpha_1$-antitrypsin.

Figure 1

Column I: Affi-Gel Blue I
Column II: Affi-Gel Blue II
Column III: DEAE-Cellulose
B: Buffer
V: Valve
F: Fraction Collector

EP 0 436 086 A1

# ISOLATION OF $\alpha_1$-ANTITRYPSIN FROM HUMAN PLASMA AND CONSTRUCTION OF A KIT USEFUL FOR DETECTING $\alpha_1$-ANTITRYPSIN LEVELS IN BLOOD

## BACKGROUND OF THE INVENTION

### 1. Field of the Invention

The present invention relates to a process for the purification of human $\alpha_1$-antitrypsin, a potential biochemical tumor marker for hepatocellular carcinoma, and to construct a kit useful for detecting serum level of $\alpha_1$-antitrypsin.

### 2. Description of the Prior Art

Tumor markers are becoming more important as early diagnostic screening methods of malignancy in conjunction with radiological modalities. In addition to $\alpha$-fetoprotein, $\alpha_1$-antitrypsin is being considered as a new biochemical tumor marker for detecting hepatocellular carcinoma. Thus an efficient procedure for purifying homogeneous form of $\alpha_1$-antitrypsin and a kit which can be used easily for measuring levels of serum $\alpha_1$-antitrypsin should be developed. When developing a purification procedure, the following should be considered; 1) The whole process should be simple and can be finished in a short period of time, 2) The steps are carefully designed so that the protein will not be denatured during the purification process. The following methods for isolating $\alpha_1$-antitrypsin developed earlier are inefficient and the proteins isolated under the methods are considered to contain impurities.

For the isolation of serum proteins, polyacrylamide (Anal. Biochem., 21: 57-67, 1967), agarose(Scand. J. Clin. Lab. Invest., 124: 71-82, 1972) and SDS-polyacrylamide (Anal. Biochem., 109: 76-86, 1980) gels were used. However these methods have been applied just to analytical purposes due to the difficulties in recovering the proteins in intact form from those gels. To circumvent this problem, people were using various columns for the purification of serum proteins. For example, Crawfort et al. (Arch. Biochem. & Biophys., 156: 215-222, 1973) used DEAE-cellulose, QAE-Sephadex and Sephadex 150 columns and Saklatvala(Biochem. J., 157: 339-351, 1976) used DEAE-cellulose and ConA-Sepharose columns for the purification of $\alpha_1$-antitrypsin. Affi-Gel Blue Sepharose together with other ion exchangers and molecular sieve columns was also used by Travis(Med. Enz. 80: 754-765, 1981) and Kang (Kor. Biochem. J., 21:485-490, 1988) to purify $\alpha_1$-antitrypsin. However these procedures are rather complicated and time consuming with increased chances for a reduction of the purification yield of the enzyme.

In general, when developing a procedure for detecting levels of certain protein, the following should be taken into consideration;1) The measurement should be accurate. 2) The procedure should be simple, easy to handle and take a short period of time. 3) If it is possible, its better not to use any harmful chemicals, dangerous machines or expensive tools. In this respect, the procedures using radial immunodiffusion (RID) method used previously by Ouchterlony (In Progress in Allergy, 5: 1, 1958), Lieberman (Chest., 62: 557, 1972), Kew (Br. J. Cancer, 37: 635, 1978), Stockley (Am. Rev. Respiratory Dis., 120: 1981), Fragion (Clin. Genet., 19: 134, 1981), Kim (Kor. J. Internal Med., 24: 222, 1981), and Sparos (Br J. Cancer, 49: 567, 1984) are inefficient since the method is not sensitive enough to measure one tenth of a $\mu$g of certain serum protein. Rocket immunoelectrophoresis (RIE) in sodium barbital buffer, however, produces reliable numbers of serum protein level. By using RIE, Laurell (Anal. Biochem., 15: 45, 1966) Eriksson(Acta Med. Scand., 195: 451, 1974), Vaughan(B.B.A., 701: 339, 1982), Choi (Cancer, 43: 596, 1979), and Bernacka(Cancer, 62: 1188, 1988) detected the level of $\alpha_1$-antitrypsin and other proteins. Gaidulis(Clin. Chem., 29(10): 1983) and Carlson(Scand. J. Gastroenterol., 20: 835, 1985) also measured the serum level of $\alpha_1$-antitrypsin through nephelometric and ELISA methods, respectively. As explained above it is true that most of the procedures previously reported employed RID or RIE. But these two methods have their own disadvantages. RID is rather time consumming (>48 hrs) and creates only approximate values. RIE has shorter assay time (3-6 hrs) but needs a cooling system (Anal. Biochem., 15: 45, 1966) for electrophoresis and requires barbital for the buffer which is a habituational and addictive drug.

## BRIEF DESCRIPTION OF THE DRAWINGS

The invention will be illustrated below by means of the accompanying drawings, wherein:

Fig. 1    shows the connected columns used for the purification of human $\alpha_1$-antitrypsin.

Fig. 2    shows the results of rocket immunoelectrophoresis in 40 mM Tris-Acetate buffer (pH 8.0) containing 1mM EDTA.

SUMMARY OF THE INVENTION

It is an object of the present invention to develop an efficient procedure for the purification of human $\alpha_1$-anti-trypsin and to provide a kit through which the serum $\alpha_1$-antitrypsin level can be detected without using habituational and addictive drugs or expensive instruments such as ELISA reader.

For the purification of $\alpha_1$-antitrypsin in homogeneous form, agarose or polyacrylamide gel electrophoresis was performed followed by a series of column chromatography. Directly connected Affi-Gel Blue and DEAE-cellulose columns produced homogeneous form of $\alpha_1$-antitrypsin in a short period of time.

For the detection of serum $\alpha_1$-antitrypsin levels in a simple and fast way, the procedure of RIE (17) has been modified. Instead of barbital, Tris-Acetate buffer was used for electrophoresis. The modified method does not require a cooling system and can be performed in room temperature.

The actual measuring distance (rocket height of the gel) in RIE experiment is usually near 100 mm while the diameter of the diffused area in RID experiment is shorter than 10 mm. Therefore it is true that RIE produces more accurate values of serum level of proteins than RID. The differences between RID and RIE are summarized in Table 1.

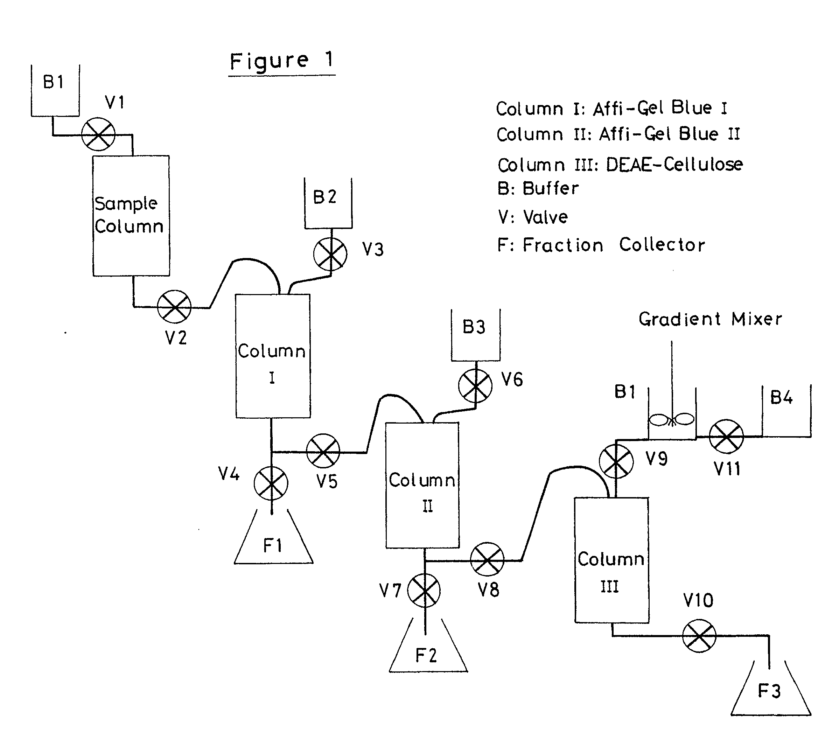

The following is the outline of the procedure for the purification of human $\alpha_1$-antitrypsin. Human serum was chilled to 0-4°C. Ammonium sulfate was added to the serum to reach the final of 50% saturation. After standing the sample at 4°C for 30-60min, the solution was centrifuged and the supernatant was recovered. Additional ammonium sulfate was added to the supernatant until the final concentration reached 80% saturation. Again, this sample was stood at 4°C for 30-60 min and centrifuged. The precipitate was then resuspended and dialyzed against TAE (40 mM Tris-acetate, pH 8.0, 1mM EDTA) or phosphate(20mM sodium phosphate, pH 8.0, 1mM EDTA) buffer. Sample protein was subjected to electrophoresis on 0.9-1.5% agarose or 8-12% polyacrylamide gel. After electrophoresis the protein band which was run together with xylene cyanol was cut out and powdered in mortar under liquid nitrogen. Steps are further illustrated in Fig. 1. The frozen powder was transferred to the sample column. The powdered gel was thawed by adding TAE buffer which had been kept at 4-8°C. Columns I and II were packed with Affi-Gel Blue and column III was filled with DEAE-cellulose Proteins eluted from the gel was passed sequentially through these three connected columns. The buffer used here contained 10-50mM Tris, pH 6.5-8.5, 1mM EDTA, and 1mM $\beta$-mercaptoethanol. When the buffer was collected in F3 (Fig. 1) up to 10 times of the bed volume of sample column, valve 8 was closed. 1-10ml fractions were collected. Optical density of each fraction was measured at 280nm. The fractions containing $\alpha_1$-antitrypsin were pooled.

The method explained above is a new technique which is not known to the public, and is expected to be widely used for purifying $\alpha_1$-antitrypsin and other proteins. Actual purification works are detailed further in Example 1) and the purity of the product and the time required for the experiment are summarized in Table 2 and 3, respectively, with those of known methods.

About 100-150 $\mu$g each of the homogeneous $\alpha_1$-antitrypsin purified by the experiment explained above was injected into each rabbit. A monospecific antiserum for $\alpha_1$-antitrypsin was obtained. The antiserum was added to the agarose solution (50-100mM Tris-Acetate, pH 8.0, 2mM EDTA, 0.8-1.2% agarose) at 45-55°C to make the final concentration of 0.5-3.0%. The agarose solution containing the antiserum was poured onto the gel plate. Horizontal gel electrophoresis in 50-100mM Tris-Acetate buffer(pH 8.0) containing 2mM EDTA was performed for 1-3 hours at 10-20 volts/cm. After the electrophoresis, the serum level of $\alpha_1$-antitrypsin was calculated by comparing the rocket height with that of known amount of standard samples. The actual process performed in this experiment is explained further in Example 2). The procedure mentioned above has advantages on the following respects; 1) The serum $\alpha_1$-antitrypsin level can be measured accurately. 2) By using Tris-acetate buffer instead of barbital buffer, a habituational and addictive drug, the electrophoresis could be handled in normal way. 3) The electrophoresis can be performed at room temperature without any cooling system and finished within 3 hours.

EXAMPLE

Example 1

Isolation of $\alpha_1$-Antitrypsin from human plasma in homogeneous form by rocket immunoelectrophoresis in Tris-Acetate buffer and three connected columns of Affi-Gel Blue and DEAE-Cellulose

Powdered ammonium sulfate was added to the human serum until the concentration reached 50% saturation. After standing it at 4°C for 60min, the supernatant was recovered after centrifugation. Additional powdered ammonium sulfate was added to the supernatant to make the final concentration of 80%. After additional standing at 4°C for 60min, the precipitate was recovered and resuspended in a buffer (40mM Tris-acetate, pH 8.0, 2mM EDTA). The sample was dialyzed against the same buffer. The protein solution was applied on a 1% agarose gel and subjected to electrophoresis for 3 hours. The band which was run together with xylene cyanole was cut out and ground in a mortar under liquid nitrogen. The frozen gel powder was placed in the sample column as illustrated in Fig. 1. The buffer (20mM Tris-HCl, pH 7.4, 1mM EDTA, 1mM $\beta$-mercaptoethanol) was then applied to the sample column to pass through Affi-Gel blue I, Affi-Gel II, and DEAE-cellulose columns. After collecting enough volumes of the buffer in F3, a linear NaCl gradient(0.0-0.3M) was applied to the DEAE-cellulose column. The result of this experiment is summarized in Table 2. The final yield was 62% and the specific activity of the purified $\propto_1$-antitrypsin was 817 units/mg. 1unit of the enzyme represents the amount of the protein which can reduce the $O.D_{410}$ of Succinyl-L-alanyl-L-alanyl-L-alanyl-L-alanyl-p-nitroanilide by 1.0 per min.

### Example 2

Detection of $\propto_1$-antitrypsin by rocket immunoelectrophoresis in Tris-Acetate buffer

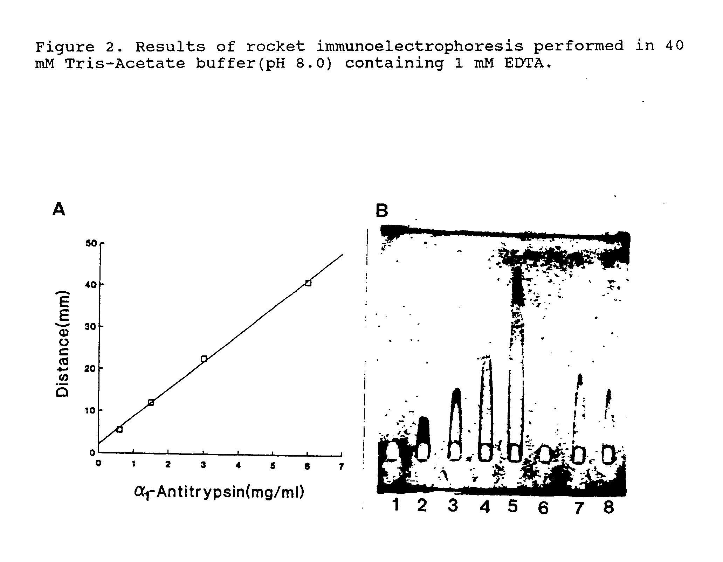

100-150 $\mu$g of the purified $\propto_1$-antitrypsin was injected into rabbit every 10th day. After 4th injection the antiserum was recovered. The antiserum was added to a agarose solution (1%) at 50°C to make the final antiserum concentration of 1.5%. The agarose solution was then poured onto the gel plate to make 3mm thick gels. When the gel was cooled down, 1 $\mu$1 each of the sample sera was applied on each hole of the gel. Electrophoresis was done in 40mM Tris-Acetate buffer(pH 8.0) containing 2mM EDTA at room temperature for 3 hours at 15 volts/cm. The rocket heights were measured and compared with that of known standard samples as shown in Fig. 2.

Figure 2 shows the results of rocket immunoelectrophoresis performed in 40mM tris-acetate buffer (pH 8.0) containing 1mM EDTA.

Purified human $\alpha_1$-antitrypsin and healthy human sera were run on a 1% agarose gel containing 1.5% rabbit antiserum in TAE (40mM tris-acetate, 1mM EDTA, pH 8.0) buffer for 3 hours at 15 volt/cm and room temperature. The gel was stained with Amido Black 10B.

A: The figure shows the relationship between the rocket height and the $\alpha_1$-antitrypsin concentration.

B: Wells 2, 3, 4, and 5 contained 0.6, 1.5, 3.0, and 6.0 $\mu$g of $\alpha_1$-antitrypsin. Wells 1 and 6 contained only TAE buffer. Wells 7 and 8 contained 1 $\mu$l of sera of healty blood donors.

Table 1. Comparison between radial immunodiffusion(RID)

and rocket immunoelectrophoresis(RIE)

|  | RID | RIE |
|---|---|---|
| Measuring time(hr) | 18-45 | 1-3 |
| Measuring subject | Diameter | Rocket height |

Table 2. Purification of $\alpha_1$-antitrypsin by the procedure

employed in the present invention

|  | Volume (ml) | Total unit* | Total protein (mg) | Specific activity (units/mg) | Yield (%) |
|---|---|---|---|---|---|
| Serum | 100 | 16,600 | 3,150 | 5.3 | 100 |
| Ammonium sulfate precipitation | 39 | 15,300 | 1,640 | 9.3 | 92 |
| Gel elution & Affi-Gel Blue & DEAE cellulose column chromatography | 35 | 10,300 | 12.6 | 817 | 62 |

* One unit of elastase-inhibitory activity was defined as
the amount of the protein which can reduce the $\Delta A_{410}$
by 1.0 absorbance units per minute. The assay mixture
contained 0.2M Tris-Cl, pH 8.0, $1.2 \times 10^{-6}$ mole of
elastase and $9.97 \times 10^{-4}$ mole of succinyl-L-alanyl-L-
alanyl-L-alanyl-L-alanyl-p-nitroanilide.

Table 3. Time required for each step in purifying $\alpha_1$-antitrypsin

| Conventional method | Time(hr) | Procedure employed in the present invention | Time(hr) |
|---|---|---|---|
| Ammonium sulfate fractionation | 2 | Ammonium sulfate fractionation | 2 |
| Dialysis | 3 | Dialysis | 3 |
| QAE-Sephadex A-50 | 5 | Gel electrophoresis | 2 |
| Ammonium sulfate precipitation | 1 | Gel elution and continuous column chromatography | 5 |
| Dialysis | 3 | | |
| Affi-Gel Blue I | 2 | | |
| Affi-Gel Blue II | 2 | | |
| Ammonium sulfate precipitation | 1 | | |
| Dialysis | 3 | | |
| Coneanavalin A column chromato-graphy | 5 | | |
| Total | 27 | Total | 12 |

**Claims**

1. The method for purifying homogeneous form of $\alpha_1$-antitrypsin (AAT) using the three connected columns (Affi-Gel Blue I, Affi-Gel II, and DEAE-cellulose) after purifying it partially through ammonium sulfate fractionation(between 50 and 75%) and agarose gel electrophoresis.

2. A kit for measuring levels of serum $\alpha_1$-antitrypsin which uses agarose gel containing 1-3% of antiserum against $\alpha_1$ -antitrypsin and is designed to perform rocket immunoelectrophoresis at room temperature in TAE buffer (100mM Tris-acetate, pH 8.0, 1mM EDTA) or other buffers which does not contain any habituational and addictive drug.

## Figure 1

Column I: Affi-Gel Blue I
Column II: Affi-Gel Blue II
Column III: DEAE-Cellulose
B: Buffer
V: Valve
F: Fraction Collector

Gradient Mixer

B1    Sample Column    V1
V2
B2    V3
Column I
B3    V6
V4    V5    Column II
F1    B1    V9    B4    V11
V7    V8    Column III
F2    V10
F3

EP 0 436 086 A1

Figure 2. Results of rocket immunoelectrophoresis performed in 40 mM Tris-Acetate buffer(pH 8.0) containing 1 mM EDTA.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 104, no. 15, 14th April 1986, page 306, abstract no. 125514t, Columbus, Ohio, US; X. GU et al.: "The isolation of highly purified alpha1-antitrypsin and preparation of its antibody for quantitative measurement of alpha1-antitrypsin by rocket immunoelectrophoresis", & SHENGWU HUAXUE YU SHENGWU WULI JINZHAN 1985, 65, 59-64 * Abstract * | 2 | C 07 K 13/00 C 07 K 15/00 G 01 N 33/574 |
| X | CHEMICAL ABSTRACTS, vol. 87, no. 23, 5th December 1977, page 209, abstract no. 179568n, Columbus, Ohio, US; D.T. FORMAN: "Measurement of serum alpha-1 anti-trypsin", & MAN. PROCED. SEMIN. CLIN. ENZYMOL. 1976, 216-25 * Abstract * | 2 | |
| X | CHEMICAL ABSTRACTS, vol. 86, no. 5, 31st January 1977, page 247, abstract no. 28357w, Columbus, Ohio, US; W. GROC et al.: "Investigation of the apparent delay in mobility of albumin, alpha-1-antitrypsin, haptoglobin 1-1 and fibrinogen in electroimmunodiffusion", & J. IMMUNOL. METHODS 1976, 12(3-4), 227-36 * Abstract * | 2 | |
| A | FR-A-2 558 961 (CNRS) * Whole document * | 1 | TECHNICAL FIELDS SEARCHED (Int. Cl.5) C 07 K G 01 N |
| A | JOURNAL OF CHROMATOGRAPHY, vol. 296, 1984, pages 221-229, Elsevier Science Publishers B.V., Amsterdam, NL; G. GUNZER et al.: "Purification of alpha1-proteinase inhibi-tor by triazine dye affinity chromatography, ion-exchange chromatography and gel filtration on fractogel TSK" * Whole document * | 1 | |

$-/-$

The present search report has been drawn up for all claims

| Place of search | Date of completion of search | Examiner |
|---|---|---|
| The Hague | 29 March 91 | MASTURZO P. |

CATEGORY OF CITED DOCUMENTS

X : particularly relevant if taken alone
Y : particularly relevant if combined with another document of the same catagory
A : technological background
O : non-written disclosure
P : intermediate document
T : theory or principle underlying the invention

E : earlier patent document, but published on, or after the filing date
D : document cited in the application
L : document cited for other reasons

&: member of the same patent family, corresponding document

European
Patent Office

**EUROPEAN SEARCH
REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A | PROTIDES BIOL. FLUIDS, vol. 33, 1985, pages 161-164; P. CHUCHANA et al.: "Purification of active human alpha1-antitrypsin from genetically engineered micro-organisms"<br>* Whole document * | 1 | |
| A | JOURNAL OF CHROMATOGRAPHY, vol. 235, 1982, pages 237-248, Elsevier Scientific Publishing Co., Amsterdam, NL; G. BIRKENMEIER et al.: "Application of dye-ligand chromatography to the isolation of alpha-1-proteinase inhibitor and alpha-1-acid glycoprotein"<br>* Whole document * | 1 | |
| | | | **TECHNICAL FIELDS SEARCHED (Int. Cl.5)** |

The present search report has been drawn up for all claims

| Place of search | Date of completion of search | Examiner |
|---|---|---|
| The Hague | 29 March 91 | MASTURZO P. |

CATEGORY OF CITED DOCUMENTS

X : particularly relevant if taken alone
Y : particularly relevant if combined with another
   document of the same catagory
A : technological background
O : non-written disclosure
P : intermediate document
T : theory or principle underlying the invention

E : earlier patent document, but published on, or after
   the filing date
D : document cited in the application
L : document cited for other reasons

 
 

& : member of the same patent family, corresponding
   document